# EUROPÄISCHE PATENTSCHRIFT

(11) **EP 0 908 439 B1**
(45) Veröffentlichungstag und Bekanntmachung des Hinweises auf die Patenterteilung: **02.01.2003**
(21) Anmeldenummer: 98117806.4
(22) Anmeldetag: 19.09.1998
(51) Int. Cl.: C07C 33/20, C07D 317/50, A61K 7/46

(54) **Die Verwendung substituierter 5-Phenyl-pentanole als Aroma- und Riechstoffe**
Use of substituted 5-phenyl-pentanols as flavour and perfume
Utilisation des 5-phényle-pentanols substitués comme matière odoriférente et parfum

(30) Priorität: 02.10.1997 DE 19743718
(43) Veröffentlichungstag der Anmeldung: 14.04.1999
(73) Patentinhaber: HAARMANN & REIMER GMBH, D-37601 Holzminden (DE)
(72) Erfinder: Esser, Peter Dr., 37603 Holzminden (DE); Rabenhorst, Jürgen Dr., 37671 Höxter (DE); Sonnenberg, Steffen Dr., 37603 Holzminden (DE); Walther, Lutz Dr., 37639 Bevern (DE)
(74) Vertreter: Mann, Volker, Dr.

(56) Entgegenhaltungen:
- EP-A- 0 009 540
- US-A- 4 512 918

## Beschreibung

Die vorliegende Erfindung betrifft die Verwendung substituierter 5-Phenyl-1-pentanole als Aroma- und Riechstoffe, Fixateure und Booster, neue substituierte 5-Phenyl-1-pentanole und einige Verfahren zu ihrer Herstellung.

Die wechselnden Trends modischer Geschmacksrichtungen führen nicht nur zu einem steigenden Bedarf an neuen Riechstoffen mit interessanten Duftnoten. Dringend gesucht werden vielmehr auch neue Riechstoffe mit zusätzlichen Eigenschaften. Von großem Interesse für die parfumistische Komposition sind Riechstoffe, die die Haftfestigkeit der Komposition verbessern (also als Fixateure wirken) oder die Stärke der geruchlichen Wahrnehmung erhöhen (also als Booster fungieren). Bei dermaßen funktionalen Riechstoffen wünscht man sich, daß sie nicht zu stark duften und keinen ausgeprägten eigenen Charakter haben, was ihnen eine möglichst breite Einsetzbarkeit in parfumistischen Kompositionen sichert.

Die bekannten Riechstoffen vom 5-Phenyl-pentanol-Typ, nämlich 5-Phenyl-pentanol, 2-Methyl-5-phenyl-pentanol (®Rosaphen) und 3-Methyl-5-phenyl-pentanol (®Phenoxanol), weisen alle ein blumiges bis rosiges Geruchsprofil auf. Bei einer eher mäßigen Haftung kann von Fixateureigenschaften nicht gesprochen werden. Neben ihren blumig-rosigen Beiträgen zu einer Riechstoffkomposition zeigen sie keine weiteren Effekte - auch nicht die oben angesprochene Boosterwirkung.

Überraschenderweise wurde gefunden, daß bestimmte substituierte 5-Phenyl-1-pentanole ausgezeichnete Eigenschaften als Fixateure und/oder Booster besitzen.

Gegenstand der Erfindung ist die Verwendung von Verbindungen der Formel 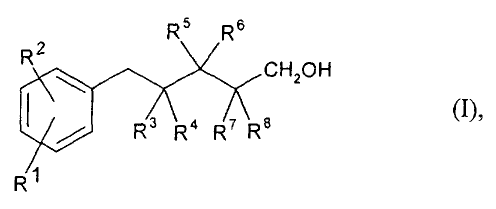 worin
- R¹, R²: unabhängig voneinander Wasserstoff, C₃-C₈-Alkyl, C₁-C₈-Alkoxy oder
- R¹ und R²: zusammen Methylendioxo -O-CH₂-O-,
- R³-R⁸: unabhängig voneinander Wasserstoff oder C₁-C₈-Alkyl
bedeuten mit der Maßgabe, daß die Gesamtsumme der Kohlenstoffatome der Substituenten R¹ - R⁸ 2 bis 8 beträgt und wenigstens ein Kohlenstoffatom auf die Reste R³-R⁸ entfällt,
als Aroma- und Riechstoffe.

Vorzugsweise werden solche Verbindungen I verwendet, worin
- R¹ und R²: die oben angegebenen Bedeutungen besitzen,
- R⁴, R⁶, R⁷: Wasserstoff und
- R³, R⁵, R⁸: unabhängig voneinander Wasserstoff oder C₁-C₈-Alkyl bedeuten
mit der Maßgabe, daß die Gesamtsumme der Kohlenstoffatome der Substituenten R¹ - R⁸ 3 bis 8 beträgt und wenigstens ein Kohlenstoffatom auf die Reste R³-R⁸ entfällt.

Verbindungen der Formel (I) sind neu mit Ausnahme von 5-Phenyl-2,4-dimethylpentanol (J. Amer. Chem. Soc. 1997, **119,** 6496; Synlett 1997, 457), 5-(4-Methoxyphenyl)-2-methyl-pentanol (J. Org. Chem. 1972, **37,** 825), 5-Phenyl-2-ethyl-pentanol (J. Amer. Chem. Soc. 1934, **56,** 689), 5-(4-Methoxy-phenyl)-3,3-dimethyl-pentanol (J. Chem. Soc., Perkin Trans. I, 1974, 394) und 5-Phenyl-3,3-dimethyl-pentanol (Chem. Ber. 1957, **90,** 1946).

Weiterer Gegenstand der Erfindung sind daher Verbindungen der obigen Formel I, ausgenommen die im vorigen Absatz namentlich aufgezählten Verbindungen, sowie Parfumöle und Aromakompositionen mit einem Gehalt an Verbindungen I.

Die Erfindung umfaßt alle möglichen Isomeren (Enantiomere, Diastereomere) und bezieht sich auf alle Verbindungen, die die erfindungsgemäßen Verbindungen (I) in gebundener, chemischer oder physikalischer Form enthalten und unter geeigneten Bedingungen freisetzen.

Die Verbindungen (I) setzen sich mit ihrem Eigenschaftsprofil als schwache, funktionale Riechstoffe überraschend deutlich von den oben genannten Riechstoffen 5-Phenyl-pentanol, 2-Methyl-5-phenyl-pentanol (Rosaphen®) und 3-Methyl-5-phenylpentanol (Phenoxanol®) ab.

Die Riechstoffe (I) sind in der parfumistischen Einzelbewertung durchgängig schwache Riechstoffe mit blumigen bis rosigen Aspekten. Ihre Stärken zeigen sie in der parfumistischen Komposition. Hier sorgen sie für einen blumig- weichen Unterton. Vor allem aber zeigen sie zwei weitere äußerst bemerkenswerte Aspekte: Die Haftung einer parfumistischen Komposition wird durch die Verbindungen (I) verbessert, das heißt sie wirken als Fixateure; weiterhin ist bei Anwesenheit von Verbindungen (I) in einer parfumistischen Komposition die geruchliche Wahrnehmung stärker, d.h. es liegt ein Booster- Effekt vor.

Die Verbindungen I können analog zu an sich bekannten Verfahren hergestellt werden.

Substituierte 2-Alkyl-5-phenyl-1-pentanole kann man herstellen, indem man z.B. Benzaldehyde der Formel R¹R²PhCHO, worin die Reste R¹ und R² die oben angegebenen Bedeutungen besitzen und Ph für Phenylen steht, in Aldolreaktionen nacheinander mit Acetaldehyd und mit Aldehyden der Formel R⁷R⁸-CH-CHO, worin R⁷ und R⁸ die oben angegebene Bedeutung besitzt, umsetzt und anschließend hydriert.

Substituierte 2,4-Dialkyl-5-phenyl-1-pentanole erhält man über doppelte Aldolreaktion der Benzaldehyde R¹R²PhCHO, worin die Reste R¹ und R² die oben angegebenen Bedeutungen besitzen und Ph für Phenylen steht, mit Aldehyden der Formeln R³R⁴-CH-CHO bzw. R⁷R⁸-CH-CHO, worin R³, R⁴, R⁷ und R⁸ die oben angegebene Bedeutung besitzt, und anschließende Hydrierung.

Eine Alternative ist die Aldolreaktion von 2-Methyl-5-phenyl-1-propanalen R¹R²PhCH₂CH(CH₃)CHO, worin die Reste R¹ und R² die oben angegebenen Bedeutungen besitzen und Ph für Phenylen steht, mit Aldehyden der Formel R⁷R⁸-CH-CHO, worin R⁷ und R⁸ die oben angegebene Bedeutung besitzt, und anschließende Hydrierung des Reaktionsprodukts.

2-Alkyl-5-phenyl-1-pentanole erhält man, indem man Zimtaldehyde der Formel 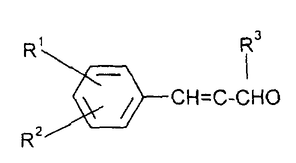 worin
- R¹- R³: die in oben angegebenen Bedeutungen besitzen,
mit Aldehyden der Formel R⁷R⁸-CH-CHO, worin R⁷ und R⁸ die oben angegebene Bedeutung besitzt, in einer Aldolreaktion reagieren läßt und anschließend hydriert.

Die Durchfürung der Aldolkondensationen kann auf an sich bekannte Weise erfolgen, z.B. nach Organikum, 19. Aufl., Barth Verlagsgesellschaft, Leipzig 1993, Seiten 467 ff., Org. React. 1968, 16, 1-438 oder Methoden der Organischen Chemie (Houben-Weyl), Bd. 21 b, Thieme Verlag, Stuttgart 1995, 1603 ff.. Als Lösungsmittel werden bevorzugt Alkohole wie Methanol oder Ethanol eingesetzt, in denen der basische Katalysator, vorzugsweise ein Alkalimetallhydroxid, wie z.B. Natrium- und/oder Kaliumhydroxid, gelöst wird.

Die Reaktionstemperatur beträgt in der Regel -20 bis 50, vorzugsweise 0 bis 30°C. Bei dieser Temperatur dosiert man die beiden Aldehyde zu. Das Molverhältnis des höhermolekularen zum niedermolekularen Aldehyds wird in der Regel 1 bis 3, vorzugsweise 1,1 bis 2 betragen. Die Zugabe der Aldehyde kann unverdünnt oder gelöst in einem Lösungsmittel, wie z.B. in Alkoholen oder in Wasser, erfolgen.

Ist eine der beiden Aldehydkomponenten Acetaldehyd, kann es vorteilhaft sein, die andere Aldehydkomponente zusammen mit der alkalischen Lösung vorzulegen und den Acetaldehyd in Lösungsmitteln wie Alkoholen oder Wasser, vorzugsweise in Wasser gelöst, zuzugeben. Die Nachreaktion kann 1 bis 48, vorzugsweise 3 bis 24 Stunden betragen.

Zur Aufarbeitung kann man die Reaktionslösung zunächst neutralisieren, wofür beispielsweise Eisessig verwendet werden kann. Dann kann man das Lösungsmittel, gegebenenfalls unter vermindertem Druck, abdestillieren.

Der Destillationsrückstand kann mit Wasser und einem organischen Lösungsmittel aufgenommen werden. Als organische Lösungsmittel können aromatische Kohlenwasserstoffe, wie z.B. Toluol, oder Ether oder Ester eingesetzt werden. Die organische Phase kann dann destilliert werden. Fällt das Reaktionsprodukt aus der Reaktionslösung aus, so kann nach Neutralisation des Reaktionsgemischs das Reaktionsprodukt abfiltriert und durch Umkristallisation gereinigt werden.

Der nach einer oder nach zwei Aldolkondensationen erhaltene ungesättigte Aldehyd kann in Substanz oder in einem alkoholischen Lösungsmittel, wie beispielsweise Methanol, Ethanol oder Isopropanol, hydriert werden. Als Hydrierkatalysatoren können handelsübliche Katalysatoren wie Raney-Nickel, Palladium/Kohle oder Platin/Kohle verwendet werden. Es können Drücke von im allgemeinen 1 bis 30, vorzugsweise 5 bis 20, insbesondere 8 bis 15 bar und Hydriertemperaturen von 20 bis 120, vorzugsweise von 40 bis 100°C gewählt werden.

Am Phenylring unterschiedlich substituierte 5-Phenyl-1-pentanole lassen sich analog zu bekannten Umsetzungen auch durch Friedel-Crafts-Alkylierung von 5-Phenyl-1-pentanolen erhalten.

Vor der Alkylierung schützt man vorteilhafterweise die Alkoholgruppe, z.B. durch Veresterung zum Acetat. Hierzu kann man das Phenylpentanol gemeinsam mit Acetanhydrid (Molverhältnis in der Regel 1 bis 2, vorzugsweise 1 bis 1,3) vorlegen. Bei einer Reaktionstemperatur von in der Regel 80 bis 120, vorzugsweise 90 bis 110°C und reduziertem Druck destilliert man zunächst Essigsäure ab.

Der Phenylpentylester kann zur Alkylierung in einem Alkan, beispielsweise Hexan oder Heptan, einem aromatischen Kohlenwasserstoff, beispielsweise Toluol oder Xylol, einem Cycloalkan, beispielsweise Cyclohexan oder Methylcyclohexan vorgelegt werden.

Als Katalysator kommen Lewis-Säuren, beispielsweise Aluminium(III)-chlorid, Titantetrachlorid oder Eisen(III)-chlorid in Betracht.

Bei einer Temperatur von 40 bis 100, vorzugsweise 40 bis 60°C gibt man das Olefin zu. Das Molverhältnis Phenylpentylester/Olefin wird in der Regel 1 bis 3, vorzugsweise 1,1 bis 2 betragen.

Der Alkylphenylpentylester liefert nach Umesterung das gewünschte Alkylphenylpentanol. Zur Umesterung eignen sich niedere Alkohole wie Methanol oder Ethanol. Diese werden in einem deutlichen molaren Überschuß eingesetzt. Das Molverhältnis Alkylphenylpentylester/Alkohol wird in der Regel 2 bis 10, vorzugsweise 3 bis 5 betragen.

Als Umesterungskatalysatoren eignen sich Basen, beispielsweise Alkalimetallalkoholate wie Natriummethylat oder Natriumethylat.

Das Umesterungsgleichgewicht kann durch Abdestillieren von Ester auf die gewünschte Seite verschoben werden.

Am Phenylring unterschiedlich substituierte 3-Methyl-5-phenyl-1-pentanole lassen sich über die Stufen Prins-Reaktion und Hydrierung gewinnen. In der Prins-Reaktion werden aromatische Aldehyde R¹R²PhCHO, worin die Reste R¹ und R² die oben angegebene Bedeutung besitzen und Ph für Phenylen steht, und 3-Methyl-3-buten-1-ol miteinander zur Reaktion gebracht.

Der aromatische Aldehyd und 3-Methyl-3-buten-1-ol werden vorzugsweise äquimolar eingesetzt. Als Lösungsmittel eignen sich aromatische Kohlenwasserstoffe, beispielsweise Toluol oder Xylol, Alkane, beispielsweise Hexan oder Heptan, Cycloalkane, beispielsweise Cyclohexan oder Methylcyclohexan, oder Ether oder Ester.

Die Reaktion kann sauer katalysiert werden. Geeignete saure Katalysatoren sind aromatische Sulfonsäuren und Alkansulfonsäuren, beispielsweise p-Toluolsulfonsäure oder Methansulfonsäure, Mineralsäuren oder ihre sauren Salze, beispielsweise Schwefelsäure oder Kaliumhydrogensulfat, und saure Ionenaustauscher, wie sie beispielsweise unter den Marken ®Lewatit oder ®Amberlite erhältlich sind. Saure Ionenaustauscher sind besonders bevorzugte saure Katalysatoren.

Durch Kochen am Wasserabscheider und Abtrennen des Reaktionswassers kann das Reaktionsgleichgewicht zu den gewünschten Produkten hin verschoben werden.

Das Produkt der Prins-Reaktion kann durch Hydrierung in das gewünschte Phenylpentanol übergeführt werden. Als Hydrierkatalysatoren können die oben genannten Hydrierkatalysatoren bei Hydrierdrücken von 1 bis 30, vorzugsweise 5 bis 20, insbesondere 8 bis 12 bar bei Hydriertemperaturen von im allgemeinen 20 bis 120, vorzugsweise 40 bis 100°C gewählt werden.

Weiterer Gegenstand der Erfindung sind daher diese Verfahren zur Herstellung der neuen Verbindungen I.

**Tabelle 1:**

| Geruch der Verbindungen (I). Angegeben sind lediglich die über rosig und blumig hinausgehenden Aspekte. | | | | | | | | | |
|---|---|---|---|---|---|---|---|---|---|
| Verb .Nr. | R¹ | R² | R³ | R⁴ | R⁵ | R⁶ | R⁷ | R⁸ | Geruch |
| 1 | 4-i-Pr | H | H | H | H | H | H | Me | leicht fruchtig |
| 2 | 4-t-Bu | H | H | H | H | H | H | Me | etwas Richtung p-Kresol |
| 3 | 4-MeO | H | H | H | H | H | H | Me | etwas anisig-süßlich, wie Heliotropin |
| 4 | 3,4-Methylendioxo | H | H | H | H | H | H | Me | Dimethylresorcinat, Fettalkohol |
| 5 | 4-i-Pr | H | Me | H | H | H | H | Me | schwach |
| 6 | 4-t-Bu | H | Me | H | H | H | H | Me | |
| 7 | 4-MeO | H | Me | H | H | H | H | Me | |
| 8 | 3,4-Methylendioxo | H | Me | H | H | H | H | Me | etwas fettig, Perillaaldehyd |
| 9 | 4-i-Pr | H | H | H | Me | H | H | H | Mimose |
| 10 | 4-t-Bu | H | H | H | Me | H | H | H | schwach |
| 11 | 4-MeO | H | H | H | Me | H | H | H | Hydroxyzimtalkohol |
| 12 | 3,4-Methylendioxo | H | H | H | Me | H | H | H | p-Kresol |
| 13 | H | H | H | H | H | H | H | Et | Hydroxyzimtalkohol, Styrex |
| 14 | H | H | H | H | H | H | H | Pr | Resedaketon |
| 15 | H | H | H | H | H | H | H | i-Pr | fruchtig |
| Abkürzungen: Me = Methyl, MeO = Methoxy, Et = Ethyl, Pr = n-Propyl, i-Pr = Isopropyl, t-Bu = tert.-Butyl | | | | | | | | | |

Riechstoffkompositionen unter Zusatz der erfindungsgemäßen Verbindungen (I) zeigen eine insgesamt komplexere, parfumistischere Wirkung. Generell kann eine deutliche Steigerung der parfumistischen Haftung festgestellt werden. Darüber hinaus ist eine Booster-Wirkung zu erkennen, d.h. die geruchliche Wahrnehmung wird durch Zusätze von Verbindungen (I) deutlich gesteigert gegenüber den gleichen Riechstoffen oder Riechstoffkompositionen ohne Zusatz von Verbindungen (I). Die beschriebenen Einflüsse von Verbindungen (I) auf Riechstoffkompositionen zeigen sich besonders bei Vergleich der zeitlichen geruchlichen Veränderung in der Anwendung.

Die erfindungsgemäß zu verwendenden Verbindungen I lassen sich gut mit anderen Riechstoffen in unterschiedlichen Mengenverhältnissen zu neuartigen, interessanten Riechstoffkompositionen kombinieren, wobei die Menge 0,1 bis 60, vorzugsweise 0,5 bis 40 Gew. %, bezogen auf die ganze Komposition, beträgt.

Außer in der Feinparfumerie können derartige Kompositionen zur Parfumierung von Kosmetika wie Cremes, Lotionen, Aerosolen, Toilettenseifen, Deodorantien, Haushaltsprodukten, wie Reinigungs- und Waschmitteln, Weichspülem, Desinfektionsmitteln und Textilbehandlungsmittel dienen, wobei die Menge der Riechstoffkomposition 0,1 bis 40, vorzugsweise 0,5 bis 20 Gew.-%, bezogen auf das ganze Produkt, beträgt.

Träger für die Aroma- und Riechstoffe können Alkohole (Methanol, Ethanol, n-Propanol, iso-Propanol und die Butanole) und an sich bekannte Öle sein.

Es ist natürlich auch möglich, die erfindungsgemäßen Aroma- und Riechstoffe mit anderen Aroma- und Riechstoffen zu kombinieren; beispielsweise seien hier genannt:
tierische Riechstoffe, wie Moschuskörneröl, Zibeth, Castoreum, Ambra;
pflanzliche Riechstoffe, wie Sandelholzöl, Neroliöl, Bergamottöl, Limonen, Lavendelöl, Salbeiöl, Rosmarinöl, Pfefferminzöl, Eukalyptusöl, Verbenaöl, Citronellöl, Cajeputöl, Sapinöl, Nelkenöl, Kamillenöl, Costus, Labdanumöl, Ginsterextrakt, Broomextrakt, Karottensamenextrakt, Jasminextrakt, Mimosenextrakt, Narzissenextrakt, Olibanumextrakt, Rosenextrakt und ähnliche; und
Chemikalien wie Acetophenon, Dimethylindanderivate, Naphthalinderivate, Allylcaprate, α-Amylzimtaldehyd, Anethol, Anisaldehyd, Benzylacetat, Benzylalkohol, Benzylpropionat, Borneol, Cinnamylacetat, Zimtalkohol, Citral, Citronellal, Cuminaldehyd, Cyclamenaldehyd, Decanol, Ethylbutyrat, Ethylcaprat, Ethylcinnamat, Ethylvanillin, Eugenol, Geraniol, Hexenol, α-Hexylzimtaldehyd, Hydroxycitronellal, Indol, Isoamylacetat, Isoamylalkohol, Isovalerat, Isoeugenol, Linalool, Linalylaceat, p-Methylacetophenon, Methylanthranilat, Methyldihydrojasmonat, Methyleugenol, Methyl-β-naphthylketon, Methylphenylcarbenylacetat, Moschusketon, Moschusxylol, Phenylacetoaldehyddimethylacetat, β-Phenylethylalkohol, 3,3,5-Trimethylcyclohexanol, γ-Undecalacton, Undecenal, Vanillin und ähnliches.

Diese Riechstoffe können einzeln oder in Kombination verwendet werden.

Die Prozentangaben der nachfolgenden Beispiele beziehen sich jeweils auf das Gewicht.

### Beispiele

### Beispiel 1: 2-Methyl-5-(4-isopropylphenyl)-pentanol (1)

### 3-(4-Isopropylphenyl)-prop-2-en-1-on

Eine Lösung aus 1000 ml Methanol, 4 g Natriumhydroxid und 148 g Cuminaldehyd wird vorgelegt und auf 4°C gekühlt. Innerhalb einer Stunde erfolgt die Zugabe einer wäßrigen Acetaldehydlösung (70,6 g in 100 g Wasser), wobei die Temperatur auf bis zu 10°C ansteigt. Man läßt noch eine Stunde bei 10°C und dann über Nacht ohne weitere Kühlung rühren.

Am Morgen wird mit 6 g Eisessig neutralisiert und das Methanol im Wasserstrahlvakuum abgezogen. Es wird mit 6 g Soda, gelöst in 200 ml Wasser, und 600 ml Toluol aufgenommen. Nach Phasentrennung wird das Toluol abgezogen.

Der Ansatz ist noch dreimal wiederholt worden. Im Durchschnitt weisen die Roh-GCs 35 % Edukt und 31 % Produkt auf. Die vereinigten vier Ansätze destilliert man über eine 30 cm Füllkörperkolonne (V4A-Wendeln). Im Hauptlauf fallen bei einer Sumpftemperatur von 160-190°C und einer Kopftemperatur von 102-117°C / 0,6 mbar 161 g 3-(4-Isopropylphenyl)-prop-2-en-1-on an.

### 2-Methyl-5-(4-isopropylphenyl)-penta-2,4-dien-1-on

Zu einer auf 20°C thermostatisierten Lösung von 20 g Natriumhydroxid in 500 ml Methanol dosiert man im Verlaufe von 2 Stunden eine Lösung von 162 g p-Isopropylzimtaldehyd und 62 g Propionaldehyd in 200 ml Methanol. Der Ansatz wird über Nacht weiter gerührt.

Nach Zugabe von 31 ml Eisessig wird das Methanol im Wasserstrahlvakuum abgezogen. Der Rückstand wird mit einer Lösung von 6 g Soda in 200 ml Wasser und 300 ml Toluol aufgenommen. Die organische Phase wird destilliert. Im Hauptlauf fallen bei einer Sumpftemperatur von 180-240°C und einer Kopftemperatur von 127-140°C / 0,2 bar 107 g 2-Methyl-5-(4-isopropyl-phenyl)-penta-2,4-dien-1-on an.

### 2-Methyl-5-(4-isopropylphenyl)-pentanol (1)

Die 107 g 2-Methyl-5-(4-isopropylphenyl)-penta-2,4-dien-1-on werden mit Isopropanol auf 500 ml aufgefüllt und mit 5 % Raney-Nickel bei 10 bar Wasserstoffdruck und einer maximalen Temperatur von 100 °C hydriert.

Nach Abfiltrieren des Raney-Nickels wird über eine 30 cm Füllkörperkolonne destilliert. Es verbleiben 71,3 g 2-Methyl-5-(4-isopropylphenyl)-pentanol (1).

### Beispiel 2: 2,4-Dimethyl-5-(4-isopropylphenyl)-pentanol (5)

### 2,4-Dimethyl-5-(4-isopropylphenyl)-pent-2-en-1-on

500 ml Methanol und 20 g Natriumhydroxid werden in einem Doppelwandgefäß vorgelegt. Binnen 2 Stunden dosiert man eine Mischung von 380 g Cyclamenaldehyd und 133,4 g Propionaldehyd bei 20°C zu. Es wird über Nacht nachgerührt.

Zur Neutralisation versetzt man mit 30 g Eisessig und zieht das Methanol im Wasserstrahlvakuum ab. Man nimmt mit einer Lösung aus 6 g Soda in 200 ml Wasser und 300 ml Toluol auf, setzt eine Spatelspitze Jonol zu und engt die organische Phase im Wasserstrahlvakuum am Rotationsverdampfer ein. Anschließend wird zweimal über eine 30 cm Füllkörperkolonne fraktioniert destilliert. Nach der zweiten Destillation erhält man bei einer Sumpftemperatur bis zu 200°C und einer Kopftemperatur von 122-126°C / 0,5 mbar 102 g des 2,4-Dimethyl-5-(4-isopropylphenyl)-pent-2-en-1-ons.

### 2,4-Dimethyl-5-(4-isopropylphenyl)-pentanol (5)

102 g 2,4-Dimethyl-5-(4-isopropylphenyl)-pent-2-en-1-on werden mit Methanol auf 500 ml aufgefüllt und mit 5 % Raney-Nickel bei 10 bar Wasserstoffdruck und einer maximalen Temperatur von 100 °C hydriert.

Nach Abfiltrieren des Raney-Nickels wird über eine 30 cm Füllkörperkolonne destilliert (120°C; 0,62 mbar). Es verbleiben 58,5 g 2,4-Dimethyl-5-(4-isopropylphenyl)-pentanol (5).

### Beispiel 3: 3-Methyl-5-(4-methoxyphenyl)-pentanol (11)

### Reaktion von Anisaldehyd mit 3-Methylbutenol

Einen Ansatz aus 272,4 g Anisaldehyd, 175,3 g 3-Methylbutenol, 1000 ml Toluol und 10 g Lewatit SPC 118 (stark sauer) kocht man über Tag in einem 2 L Dreihalskolben am Wasserabscheider unter Rückfluß. 35 g Wasser kommen zur Abscheidung. Nach Abfiltrieren des Ionenaustauschers engt man im Wasserstrahlvakuum am Rotationsverdampfer ein. Unter Zusatz einer Spatelspitze lonol und 1 g Natriumhydrogencarbonat wird über eine 50 cm Füllkörperkolonne destilliert. Nach Abtrennen von Edukt erhält man bei einer Sumpftemperatur von 160°C bis 170°C und bei einer Kopftemperatur von 98°C bis 108°C / 0,16 mbar 110 g der beiden isomeren Produkte.

### 3-Methyl-5-(4-methoxyphenyl)-pentanol (11)

110 g der beiden isomeren Produkte der Vorstufe werden mit Methanol auf 500 ml aufgefüllt und mit 1 % Pd/C bei 7 bar Wasserstoffdruck und einer maximalen Temperatur von 100 °C hydriert.

Nach Abfiltrieren des Pd/C wird über eine 30 cm Füllkörperkolonne destilliert. Man erhält 55,4 g 3-Methyl-5-(4-methoxyphenyl)-pentanol (11) bei einer Kopftemperatur von 113 °C / 0,065 mbar.

### Beispiel 4: 2-Ethyl-5-phenyl-pentanol (13)

### 2-Ethyl-5-phenyl-penta-2,4-dien-1-on

In einem auf 20°C thermostatisierten Reaktionsgefäß werden 500 ml Methanol und 20 g Natriumhydroxid vorgelegt und unter Stickstoffatmosphäre und Rühren innerhalb von 4 Stunden eine Mischung aus 264 g Zimtaldehyd und 165,6 g Butanal zugetropft. Über Nacht wird der Ansatz weiter gerührt.

Es wird mit 30 g Eisessig neutralisiert und nachfolgend das Methanol im Wasserstrahlvakuum abgezogen. Man nimmt mit 300 ml Toluol auf und wäscht mit einer Lösung von 6 g Soda in 200 ml Wasser. Die organische Phase wird im Wasserstrahlvakuum am Rotationsverdampfer eingeengt. Es wird im Ölpumpenvakuum destilliert. Der Hauptlauf enthält verschiedene isomere Produkte, hat einen Siedepunkt von 111°C/0,18 mbar und wiegt 254 g.

### 2-Ethyl-5-phenyl-pentanol (13)

254 g 2-Ethyl-5-phenyl-penta-2,4-dien-1-on werden mit Methanol auf 500 ml aufgefüllt und mit 5 % Raney-Nickel bei 10 bar Wasserstoffdruck und einer maximalen Temperatur von 100 °C hydriert. Nach Abfiltrieren des Raney-Nickels wird über eine 30 cm Füllkörperkolonne destilliert (91°C; 0,038 mbar). Es verbleiben 146,9 g 2-Ethyl-5-phenyl-pentanol (13).

### Beispiel 5: 5-(4-tert-Butylphenyl)-2-methyl-1-pentanol (2)

### 2-Methyl-5-phenyl-1-pentenyl-acetat

445 g 2-Methyl-5-phenyl-pentanol und 280 g Essigsäureanhydrid werden vorgelegt. Bei einem Druck von 200 mbar heizt man auf 100°C Sumpftemperatur auf. Bei dieser Temperatur beginnt die Abspaltung von Essigsäure (exotherm, Temperaturanstieg bis 105°C). Zunächst wird Essigsäure abdestilliert. Als Hauptfraktion werden 530 g 2-Methyl-5-phenyl-1-pentylacetat erhalten (Sumpftemperatur 146-150°C, Kopftemperatur 109-112°C / 0,6 mbar).

### 5-(4-tert-Butylphenyl)-2-methyl-1-pentyl-acetat

530 g 2-Methyl-5-phenyl-1-pentylacetat werden in 1500 g Cyclohexan vorgelegt und innerhalb von 30 min portionsweise mit 420 g Eisen(III)chlorid versetzt (schwach exotherm: Temperaturanstieg von 20°C auf 35°C). Man erwärmt mit einem Wasserbad auf 50°C und leitet bei dieser Temperatur 83 L Isobuten in 50 min ein. Die Nachreaktion erfolgt für 30 min bei 50°C. Den warmen Ansatz gießt man auf ein Gemisch von 750 g Salzsäure und 1750 g Eis. Die organische Phase wäscht man nacheinander mit 500 g Wasser und 490 g Wasser; danach versetzt man mit 10 g NaOH techn. (50 %-ige Lösung). Die organische Phase wird über eine 30 cm Füllkörperkolonne bis zu Sumpftemperatur 60°C, Kopftemperatur 51-55°C / 500-100 mbar andestilliert. Der Rückstand, bei 220°C / 1 mbar Manteltemperatur über einen Dünnschichtverdampfer destilliert, ergibt 595 g 5-(4-tert-Butylphenyl)-2-methyl-1-pentyl-acetat als Destillat.

### 5-(4-tert-Butylphenyl)-2-methyl-1-pentanol (2)

Eine Mischung aus 595 g 5-(4-tert.-Butylphenyl)-2-methyl-1-pentyl-acetat, 200 g Methanol und 3,5 g Natriummethylatpulver wird bei Normaldruck langsam auf 65°C erwärmt. Bei dieser Temperatur springt die Umesterung an (exotherm, Temperaturanstieg bis 75°C, starkes Sieden), und das Azetrop Methylacetat / Methanol destilliert ab. Nach Abnahme von Methylacetat/Methanol-Vorläufen wird bei einer Sumpftemperatur von 180-200°C und einer Kopftemperatur von 131°C / 0,6 mbar eine Hauptfraktion von 430 g erhalten. Das Produkt weist nach GC-Analyse neben 89 % 5-(4-tert-Butylphenyl)-2-methyl-1-pentanol (2) auch 4,3 % 5-(3-tert-Butylphenyl)-2-methyl-1-pentanol und 1,1 % 5-(3,4-Di-tert-butylphenyl)-2-methyl-1-pentanol auf.

### Anwendungsbeispiel 1

5-(4-tert-Butylphenyl)-2-methyl-1-pentanol (Verb. 2) wird in eine florale Riechstoffkomposition eingearbeitet. Vor den einzelnen Komponenten ist in Gewichtsteilen deren Anteil an der Komposition angegeben:
- 60: Bergamot Öl farblos H&R
- 10: Methylanthranilat
- 1: Datilat H&R
- 85: Lilial Givaudan/Roure
- 120: Lyral IFF
- 30: Mugetanol H&R
- 30: Linalool
- 15: Freesiol H&R
- 40: Terpineol alpha
- 5: Geranium Öl afrikanisch
- 1: Rosenoxid L
- 4: Phenylethylphenylacetat
- 30: Phenylethylalkohol
- 20: Citronellol
- 20: Rosaphen H&R
- 7: Geranylacetat
- 20: Benzylacetat
- 100: Methyldihydrojasmonat
- 25: Ylang Ylang Öl II
- 50: Hexenylsalicylat cis-3
- 35: Benzylsalicylat
- 10: Ionon beta Givaudan/Roure
- 2: Boronal H&R
- 10: Iraldein Gamma H&R
- 7: Eugenol
- 10: Isoeugenol cryst.
- 3: Heliotropin
- 3: Vanillin
- 5: Benzylcinnamat
- 20: Acetylcedren
- 4: Boisanol H&R
- 40: Palisandal H&R
- 15: Sandolen H&R
- 1: Ambroxid H&R
- 10: CPD / Cyclopentadecanolid Supra H&R
- 40: Galaxolid 50 % IFF
- 10: Ambrettia H&R
- 2: Indoflor Cryst. H&R
- 50: Magnolan H&R
- 100: 5-(4-tert-Butylphenyl)-2-methyl-1-pentanol (Verb. 2)

Eine derartige florale Riechstoffkomposition ist z.B. für alkoholische Deospray's geeignet. Die Riechstoffkomposition ist mit dem Zusatz dieser erfindungsgemäßen Verbindung 2 deutlich blumiger und fruchtiger. Der Gesamteindruck ist parfumistischer und komplexer. Die Haftung der frischen blumigen Noten wird verlängert.

Bei Anwendung eines derartigen Deosprays auf der Haut zeigt sich der Einfluß des Zusatzes von Verbindung 2 in besonderem Maße in der zeitlichen geruchlichen Veränderung. Die Kopfnote ist weniger diffus und deutlich blumiger und fruchtiger (Booster-Effekt). Die Herznote ist blumiger, parfumistischer und komplexer (Booster-, Fixateur-Effekt). Der Fond zeigt eine längere Haftung der frischen und blumigen Noten, er ist insgesamt weicher (Fixateur-Effekt). Bemerkenswert ist, daß durch den Zusatz von Verbindung (2) im Parfümöl auch die Bildung des unangenehmen Schweißgeruchs deutlich reduziert und länger verhindert wird.

### Anwendungsbeispiel 2

5-(4-Isopropyl-phenyl)-2-methyl-1-pentanol (Verb. 1) wird in eine fruchtige Riechstoffkomposition eingearbeitet. Vor den einzelnen Komponenten ist in Gewichtsteilen deren Anteil an der Komposition angegeben:
- 4: Aldehyd C11 Undecylen
- 4: Aldehyd C12 Laurin
- 30: Dihydromyrcenol
- 60: Terpinylacetat
- 2: Mandaril H&R
- 10: Lavandin Öl grosso
- 40: Lilial Givaudan/Roure
- 10: Mugetanol H&R
- 45: Linalool
- 20: Terpineol
- 100: Rose H&R
- 10: Rosenoxid L 10 % DPG
- 15: Phenylethylphenylacetat
- 30: Benzylacetat
- 80: alpha-Hexylzimtaldehyd
- 20: Ylang künstl. H&R
- 40: Isoraldein 70 Givaudan/Roure
- 15: Isoeugenolmethylether
- 10: Anisaldehyd
- 30: Coumarin
- 200: Oryclon H&R
- 15: Herbaflorat H&R
- 30: Habanolid 50 % Firmenich
- 50: Tonalid PFW
- 80: Dipropylenglycol
- 50: Frutinat H&R
- 100: 5 -(4-Isopropylphenyl)-2-methyl-1-pentanol (Verb. 1)

Eine derartige fruchtige Riechstoffkomposition ist z.B. für Seifen geeignet. 5-(4-Isopropyl-phenyl)-2-methyl-1-pentanol (Verb. 1) verleiht dieser fruchtigen Komposition mehr blumige Fülle im Seifenstück. Nach Applikation der Seifenlösung auf die Haut ist bei der Komposition mit 5-(4-Isopropylphenyl)-2-methyl-1-pentanol eine weichere blumige Note wahrnehmbar. Weiterhin wird der gesamte Duftablauf verzögert, so daß eine deutlich längere Haftung entsteht.

### Anwendungsbeispiel 3

5-(4-tert-Butylphenyl)-3-methyl-1-pentanol (Verb. 10) wird in eine citrus-Riechstoffkomposition eingearbeitet. Vor den einzelnen Komponenten ist in Gewichtsteilen deren Anteil an der Komposition angegeben:
- 1,5: Aldehyd C11 10% DPG
- 2: Isoananat H&R
- 2: Vertocitral 10 % H&R
- 1: Floropal H&R
- 1,5: Hexenylacetat 10 %
- 235: Bergamot Öl farblos H&R
- 2: Galbanum Synthessence 10 % H&R
- 1: Indoflor kryst. 10 % H&R
- 240: Dihydromyrcenol
- 15: Tamarinia H&R
- 5: Oxania Base Firmenich
- 5: Lavendel Öl
- 1: Artemisia Öl Morokko
- 5: Estragol
- 2: Heliofolal H&R
- 10: Lyral IFF
- 10: Mugetanol H&R
- 50: Tetrahydrolinalool
- 10: Terpineol
- 20: Geranium Boubon Synthessence H&R
- 40: Phenylethylalkohol
- 5: Citronellol
- 5: Geraniol
- 30: Rosaphen H&R
- 1: Damascon alpha
- 5: Benzylacetat
- 10: Methyldihydrojasmonat
- 90: alpha-Hexylzimtaldehyde
- 3: Hexenylsalicylat cis-3
- 10: Hexylsalicylat
- 15: Isoraldein 70 Givaudan / Roure
- 1: Heliotropin
- 1: Vanillin
- 5: Oryclon spezial H&R
- 20: Boisanol H&R
- 20: Palisandal H&R
- 5: Sandolen H&R
- 15: Globalid 50 % DPG H&R
- 15: Cumarinia H&R
- 85: Claritone
- 100: 5-(4-tert-Butylphenyl)-3-methyl-1-pentanol (Verb. 10)

Eine derartige Riechstoffkomposition mit citrus-Note ist z.B. für Shampoos geeignet. Durch die Verwendung von 5-(4-tert-Butylphenyl)-3-methyl-1-pentanol (Verb. 10) wird eine Harmonisierung der frischen und transparenten Kopfnote mit der rosigen Herznote erzielt. Darüberhinaus ist wiederum eine Steigerung der Haftung der gesamten Komposition auf dem Haar wahrnehmbar.

### Anwendungsbeispiel 4

5-(4-tert-Butylphenyl)-2,4-dimethyl-1-pentanol (Verb. 6) wird in eine citrus-Riechstoffkomposition eingearbeitet. Vor den einzelnen Komponenten ist in Gewichtsteilen deren Anteil an der Komposition angegeben:
- 3: Precyclemon B IFF
- 5: Vertocitral H&R
- 8: Allylamylglycolat IFF
- 8: Isoananat H&R
- 50: Bergamot Öl farblos H&R
- 50: Dihydromyrcenol
- 15: Grapefruit Öl H&R
- 15: Lavandel Öl grosso
- 8: Eucalyptol
- 5: Artemisia Öl Marokko
- 5: Thyme Vitessence H&R
- 10: Basil Vitessence H&R
- 1: Fir Balsam Hyperessence farblos
- 3: Jasmaprunat H&R
- 70: Lilial Givaudan / Roure
- 10: Mugetanol H&R
- 10: Mimose Vitessence H&R
- 2: Nigritella Vitessence H&R
- 0,5: Damascenon Firmenich
- 3: Damascon delta Firmenich
- 80: Methyldihydrojasmonat
- 55: alpha-Hexylzimtaldehyd
- 10: Hexenylsalicylat cis-3
- 50: Hexylsalicylat
- 30: Isoraldeine 70 Givaudan / Roure
- 3: Vanillin
- 50: Agrumex HC H&R
- 30: Herbaflorat H&R
- 30: Cyclaprop IFF
- 55: Cedrylketon
- 15: Boisanol H&R
- 40: Patchouli Öl entfärbt H&R
- 30: Sandel 80 H&R
- 5: Sandolen H&R
- 5: Evernyl Givaudan / Roure
- 3: Ambroxan Henkel
- 2: Ambrettolid H&R
- 20: CPD / Cyclopentadecanolid supra H&R
- 80: Globalid 50 % DEP
- 107: Claritone
- 100: 5-(4-tert-Butylphenyl)-2,4-dimethyl-1-pentanol (Verb. 6)

Eine derartige Riechstoffkomposition mit citrus-Note ist z.B. für Wäscheweichspüler geeignet. Die Kopfnote der frischen und leichten Komposition erhält durch den Zusatz von 5-(4-tert-Butylphenyl)-2,4-dimethyl-1-pentanol (Verb. 6) eine weitere Steigerung. Darüberhinaus wird im Vergleich zur Orginalkomposition eine weitere Harmonisierung erzielt. Bei Anwendung des Parfumöls in einem Wäscheweich auf Baumwolle ist eine insgesamt gesteigerte Haftung durch den Zusatz von 5-(4-tert-Butyl-phenyl)-2,4-dimethyl-1-pentanol (Verb. 6) wahrnehmbar.

## Patentansprüche

1. Verwendung von Verbindungen der Formel 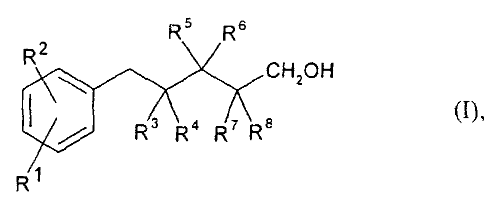 worin
R¹, R² unabhängig voneinander Wasserstoff, C₃-C₈-Alkyl, C₁-C₈-Alkoxy oder
R¹ und R² zusammen Methylendioxo -O-CH₂-O-,
R³-R⁸ unabhängig voneinander Wasserstoff oder C₁-C₈-Alkyl bedeuten mit der Maßgabe, daß die Gesamtsumme der Kohlenstoffatome der Substituenten R¹ - R⁸ 2 bis 8 beträgt und wenigstens ein Kohlenstoffatom auf die Reste R³-R⁸ entfällt,
als Aroma- und Riechstoffe.

2. Verwendung nach Anspruch 1, wobei
R¹ und R² die in Anspruch 1 angegebenen Bedeutungen besitzen,
R⁴, R⁶, R⁷ für Wasserstoff stehen und
R³, R⁵, R⁸ unabhängig voneinander Wasserstoff oder C₁-C₈-Alkyl bedeuten mit der Maßgabe, daß die Gesamtsumme der Kohlenstoffatome der Substituenten R¹ - R⁸ 3 bis 8 beträgt und wenigstens ein Kohlenstoffatom auf die Reste R³-R⁸ entfällt.

3. Verwendung nach Anspruch 1, wobei
R¹ C₃-C₈-Alkyl, C₁-C₈-Alkoxy,
R² Wasserstoff oder
R¹ und R² zusammen Methylendioxo -O-CH₂-O-,
R³-R⁷ Wasserstoff und
R⁸ Methyl bedeuten.

4. Verwendung nach Anspruch 1, wobei
R¹ C₃-C₈-Alkyl, C₁-C₈-Alkoxy,
R² Wasserstoff oder
R¹ und R² zusammen Methylendioxo -O-CH₂-O-,
R⁴-R⁷ Wasserstoff und
R³ und R⁸ Methyl bedeuten.

5. Verwendung nach Anspruch 1, wobei
R¹ C₃-C₈-Alkyl, C₁-C₈-Alkoxy,
R² Wasserstoff oder
R¹ und R² zusammen Methylendioxo -O-CH₂-O-,
R³ - R⁴ und R⁶ - R⁸ Wasserstoff und
R⁵ Methyl bedeuten.

6. Verwendung nach Ansprüchen 1 bis 5 als Fixateure und/oder Booster.

7. Verbindungen der Formel (I) nach Anspruch 1, ausgenommen
5-Phenyl-2,4-dimethyl-pentanol,
5-(4-Methoxy-phenyl)-2-methyl-pentanol,
5-Phenyl-2-ethyl-pentanol.
5-(4-Methoxy-phenyl)-3,3-dimethyl-pentanol und
5-Phenyl-3,3-dimethyl-pentanol.

8. Verbindungen nach Anspruch 7, wobei
R¹ und R² die in Anspruch 1 angegebenen Bedeutungen besitzen,
R⁴, R⁶, R⁷ für Wasserstoff stehen und
R³, R⁵, R⁸ unabhängig voneinander Wasserstoff oder C₁-C₈-Alkyl bedeuten mit der Maßgabe, daß die Gesamtsumme der Kohlenstoffatome der Substituenten R¹ - R⁸ 3 bis 8 beträgt und wenigstens ein Kohlenstoffatom auf die Reste R³-R⁸ entfällt.

9. Verfahren zur Herstellung der Verbindungen nach Ansprüchen 7 und 8 durch
a) Umsetzung von 5-Phenyl-1-pentanol-estem der Formel 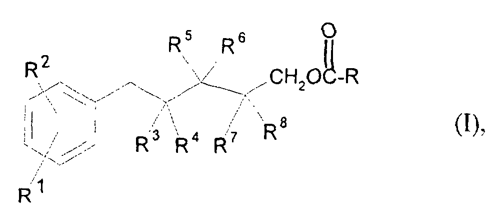 worin
R C₁-C₆-Alkyl,
R¹, R² Wasserstoff bedeuten und
R³-R⁸ die in Anspruch 1 angegebenen Bedeutungen besitzen, mit C₃-C₈-Olefinen in Gegenwart von Lewis-Säure und nachfolgende Verseifung oder
b) Umsetzung von Benzaldehyden R¹R²PhCHO, worin die Reste R¹ und R² die in Anspruch 1 angegebenen Bedeutungen besitzen und Ph für Phenylen steht, in Aldol-Reaktionen nacheinander mit Acetaldehyd und Aldehyd der Formel
R⁷R⁸CH-CHO
worin R⁷ und R⁸ die in Anspruch 1 angegebene Bedeutung besitzt,
und anschließende Hydrierung des erhaltenen Reaktionsprodukts oder
c) Umsetzung von Benzaldehyden R¹R²PhCHO, worin die Reste R¹ und R² die in Anspruch 1 angegebenen Bedeutungen besitzen und Ph für Phenylen steht, nacheinander mit je einem Äquivalent Aldehyd der Formeln
R³R⁴CH-CHO bzw. R⁷R⁸CH-CHO
worin R³, R⁴, R⁷ und R⁸ die in Anspruch 1 angegebenen Bedeutungen besitzen,
und anschließende Hydrierung des erhaltenen Reaktionsprodukts oder
d) Umsetzung von 2-Methyl-5-phenyl-1-propanalen R¹R²PhCH₂CH(CH₃)CHO, worin die Reste R¹ und R² die in Anspruch 1 angegebenen Bedeutungen besitzen und Ph für Phenylen steht, mit Aldehyden der Formel
R⁷R⁸CH-CHO
worin
R⁷ und R⁸ die in Anspruch 1 angegebene Bedeutung besitzt,
und anschließende Hydrierung des erhaltenen Reaktionsprodukts oder
e) Umsetzung von Zimtaldehyden der Formel 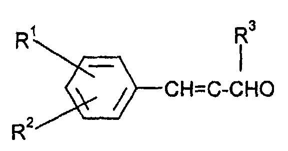 worin
R¹ - R³ die in Anspruch 1 angegebenen Bedeutungen besitzen,
mit Aldehyden der Formel
R⁷R⁸CH-CHO
worin R⁷ und R⁸ die in Anspruch 1 angegebene Bedeutung besitzt, und anschließende Hydrierung des erhaltenen Reaktionsprodukts oder
f) Umsetzung von Benzaldehyden R¹R²PhCHO, worin die Reste R¹ und R² die in Anspruch 1 angegebenen Bedeutungen besitzen und Ph für Phenylen steht, in Prins-Reaktion mit 3-Methyl-3-buten-1-ol und anschließende Hydrierung des erhaltenen Reaktionsprodukts.

10. Parfumöle oder Aromakompositionen mit einem Gehalt an Verbindungen der Formel (I) nach Anspruch 1.

11. Kosmetika mit einem Gehalt an Verbindungen der Formel (I) nach Anspruch 1.

12. Deodorantien mit einem Gehalt an Verbindungen der Formel (I) nach Anspruch 1.

13. Deodorantien mit einem Gehalt an 2-Methyl-5-(4-isopropylphenyl)-1-pentanol.

## Claims

1. Use of compounds of the formula 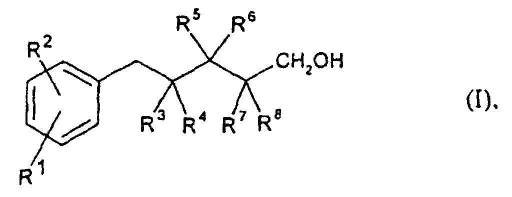 wherein
R¹, R² independently of one another denote hydrogen, C₃-C₈-alkyl, C₁-C₈-alkoxy or
R¹ and R² together denote methylenedioxo -O-CH₂-O-,
R³-R⁸ independently of one another denote hydrogen or C₁-C₈-alkyl
with the proviso that the total sum of carbon atoms of the substituents R¹ - R⁸ is 2 to 8 and at least one carbon atom falls to the radicals R³-R⁸. as flavours and perfumes.

2. Use according to claim 1, wherein
R¹ and R² have the meanings given in claim 1,
R⁴, R⁶, R⁷ represent hydrogen and
R³, R⁵, R⁸ independently of one another denote hydrogen or C₁-C₈-alkyl, with the proviso that the total sum of carbon atoms of the substituents R¹ - R⁸ is 3 to 8 and at least one carbon atom falls to the radicals R³-R⁸.

3. Use according to claim 1, wherein
R¹ denotes C₃-C₈-alkyl, C₁-C₈-alkoxy,
R² denotes hydrogen or
R¹ and R² together denote methylenedioxo -O-CH₂-O-,
R³-R⁷ denote hydrogen and
R⁸ denotes methyl.

4. Use according to claim 1, wherein
R¹ denotes C₃-C₈-alkyl, C₁-C₈-alkoxy,
R² denotes hydrogen or
R¹ and R² together denote methylenedioxo -O-CH₂-O-,
R⁴-R⁷ denote hydrogen and
R³ and R⁸ denote methyl.

5. Use according to claim 1, wherein
R¹ denotes C₃-C₈-alkyl, C₁-C₈-alkoxy,
R² denotes hydrogen or
R¹ and R² together denote methylenedioxo -O-CH₂-O-,
R³-R⁴ and R⁶-R⁸ denote hydrogen and
R⁵ denotes methyl.

6. Use according to claims 1 to 5 as fixatives and/or boosters.

7. Compounds of the formula (I) according to claim 1, excluding
5-phenyl-2,4-dimethyl-pentanol,
5-(4-methoxy-phenyl)-2-methyl-pentanol,
5-phenyl-2-ethyl-pentanol,
5-(4-methoxy-phenyl)-3,3-dimethyl-pentanol and
5-phenyl-3,3-dimethyl-pentanol.

8. Compounds according to claim 7, wherein
R¹ and R² have the meanings given in claim 1,
R⁴, R⁶, R⁷ represent hydrogen and
R³, R⁵, R⁸ independently of one another denote hydrogen or C₁-C₈-alkyl, with the proviso that the total sum of carbon atoms of the substituents R¹ - R⁸ is 3 to 8 and at least one carbon atom falls to the radicals R³-R⁸.

9. Process for the preparation of the compounds according to claims 7 and 8, by
a) reaction of 5-phenyl-1-pentanol esters of the formula 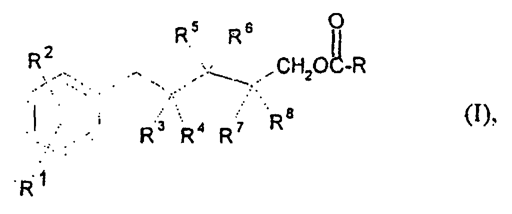 wherein
R denotes C₁-C₆-alkyl,
R¹, R² denote hydrogen and
R³-R⁸ have the meanings given in claim 1, with C₃-C₈-olefins in the presence of a Lewis acid and subsequent saponification or
b) reaction of benzaldehydes R¹R²PhCHO, wherein the radicals R¹ and R² have the meanings given in claim 1 and Ph represents phenylene, in aldol reactions successively with acetaldehyde and an aldehyde of the formula
R⁷R⁸CH-CHO
wherein R⁷ and R⁸ have the meaning given in claim 1,
and subsequent hydrogenation of the reaction product obtained or
c) reaction of benzaldehydes R¹R²PhCHO, wherein the radicals R¹ and R² have the meanings given in claim 1 and Ph represents phenylene, successively with in each case one equivalent of an aldehyde of the formulae
R³R⁴CH-CHO and R⁷R⁸CH-CHO
wherein R³, R⁴, R⁷ and R⁸ have the meanings given in claim 1,
and subsequent hydrogenation of the reaction product obtained or
d) reaction of 2-methyl-5-phenyl-1-propanals R¹R²PhCH₂CH(CH₃)CHO, wherein the radicals R¹ and R² have the meanings given in claim 1 and Ph represents phenylene, with aldehydes of the formula
R⁷R⁸CH-CHO
wherein
R⁷ and R⁸ have the meaning given in claim 1,
and subsequent hydrogenation of the reaction product obtained or
e) reaction of cinnamaldehydes of the formula 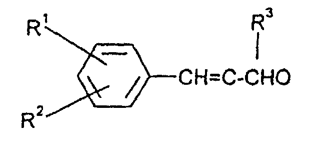 wherein
R¹-R³ have the meanings given in claim 1,
with aldehydes of the formula
R⁷R⁸ CH-CHO
wherein R⁷ and R⁸ have the meaning given in claim 1,
and subsequent hydrogenation of the reaction product obtained or
f) reaction of benzaldehydes R¹R²PhCHO, wherein the radicals R¹ and R² have the meanings given in claim 1 and Ph represents phenylene, in a Prins reaction with 3-methyl-3-buten-1-ol and subsequent hydrogenation of the reaction product obtained.

10. Perfume oils or flavour compositions with a content of compounds of the formula (I) according to claim 1.

11. Cosmetics with a content of compounds of the formula (I) according to claim 1.

12. Deodorants with a content of compounds of the formula (I) according to claim 1.

13. Deodorants with a content of 2-methyl-5-(4-isopropylphenyl)-1-pentanol.

## Revendications

1. Utilisation de composés de formule 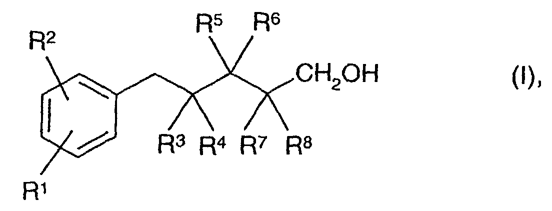 dans laquelle
R¹, R² représentent chacun, indépendamment l'un de l'autre l'hydrogène, un groupe alkyle en C₃-C₈, alcoxy en C₁-C₈,
ou bien
R¹ et R² représentent ensemble un groupe méthylènedioxo -O-CH₂-O-,
R³ à R⁸ représentent chacun, indépendamment les uns des autres, l'hydrogène ou un groupe alkyle en C₁-C₈,
sous réserve que le total des atomes de carbone des substituants R¹ à R⁸ va de 2 à 8 et qu'au moins un atome de carbone revient aux substituants R³ à R⁸,
en tant que matières aromatiques et odorantes.

2. Utilisation selon la revendication 1, pour laquelle
R¹ et R² ont les significations indiquées dans la revendication 1,
R⁴, R⁶, R⁷ représentent l'hydrogène et
R³, R⁵, R⁸ représentent chacun, indépendamment les uns des autres l'hydrogène ou un groupe alkyle en C₁-C₈,
sous réserve que le total des atomes de carbone des substituants R¹ à R⁸ va de 3 à 8 et qu'au moins un atome de carbone revient aux substituants R³ à R⁸.

3. Utilisation selon la revendication 1, pour laquelle
R¹ représente un groupe alkyle en C₃-C₈, alcoxy en C₁-C₈,
R² représente l'hydrogène ou bien
R¹ et R² représentent ensemble un groupe méthylènedioxo -O-CH₂-O-,
R³ à R⁷ représentent l'hydrogène et
R⁸ un groupe méthyle.

4. Utilisation selon la revendication 1, dans laquelle
R¹ représente un groupe alkyle en C₃-C₈, alcoxy en C₁-C₈,
R² représente l'hydrogène, ou bien
R¹ et R² représentent ensemble un groupe méthylènedioxo -O-CH₂-O-,
R⁴ à R⁷ représentent l'hydrogène et
R³ et R⁸ représentent des groupes méthyle

5. Utilisation selon la revendication 1, dans laquelle
R¹ représente un groupe alkyle en C₃-C₈, alcoxy en C₁-C₈,
R² représente l'hydrogène, ou bien
R¹ et R² représentent ensemble un groupe méthylènedioxo -O-CH₂-O-,
R³ et R⁴ et R⁶ à R⁸ représentent l'hydrogène et
R⁵ un groupe méthyle

6. Utilisation selon les revendications 1 à 5 en tant que fixateurs et/ou renforçateurs.

7. Composés de formule (I) selon la revendication 1, à l'exception des suivants :
5-phényl-2,4-diméthyl-pentanol,
5-(4-méthoxy-phényl)-2-méthyl-pentanol,
5-phényl-2-éthyl-pentanol,
5-(4-méthoxy-phényl)-3,3-diméthyl-pentanol et
5-phényl-3,3-diméthyl-pentanol.

8. Composés selon la revendication 7, pour lesquels
R¹ et R² ont les significations indiquées dans la revendication 1,
R⁴, R⁶ et R⁷ représentent l'hydrogène et
R³, R⁵ et R⁸ représentent chacun, indépendamment les uns des autres, l'hydrogène ou un groupe alkyle en C₁-C₈,
sous réserve que le total des atomes de carbone des substituants R¹ à R⁸ va de 3 à 8 et qu'au moins un atome de carbone revient aux substituants R³ à R⁸.

9. Procédé pour la préparation des composés selon les revendications 7 et 8
a) par réaction d'esters du 5-phényl-1-pentanol répondant à la formule 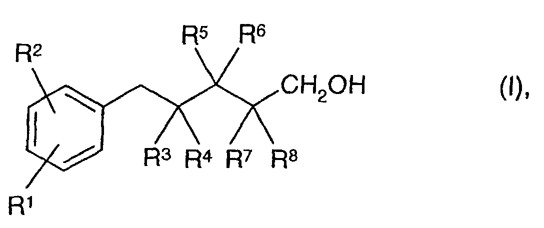 dans laquelle
R représente un groupe alkyle en C₁-C₆,
R¹ et R² représentent l'hydrogène et
R³ à R⁸ ont les significations indiquées dans la revendication 1,
avec des oléfines en C₃-C₈ en présence d'un acide de Lewis et en faisant suivre d'une saponification, ou bien
b) par réaction de benzaldéhydes R¹R²PhCHO pour lesquels R¹ et R² ont les significations indiquées dans la revendication 1 et Ph représente un groupe phénylène, dans des condensations aldoliques successives avec l'acétaldéhyde et un aldéhyde de formule
R⁷R⁸CH-CHO
dans laquelle R⁷ et R⁸ ont les significations indiquées dans la revendication 1,
et en faisant suivre d'une hydrogénation du produit de réaction ainsi obtenu, ou bien
c) par réaction de benzaldéhydes R¹R²PhCHO pour lesquels R¹ et R² ont les significations indiquées dans la revendication 1 et Ph représente un groupe phénylène, successivement avec un équivalent de chacun des aldéhydes de formules respectives
R³R⁴CH-CHO et R⁷R⁸CH-CHO
dans lesquelles R³, R⁴, R⁷ et R⁸ ont les significations indiquées dans la revendication 1,
et en faisant suivre d'une hydrogénation du produit de réaction ainsi obtenu, ou bien
d) par réaction de 2-méthyl-5-phényl-1-propanals R¹R²PhCH₂CH(CH₃)CHO, pour lesquels R¹ et R² ont les significations indiquées dans la revendication 1 et Ph représente un groupe phénylène, avec des aldéhydes de formule
R⁷R⁸CH-CHO
dans laquelle
R⁷ et R⁸ nt les significations indiquées dans la revendication 1,
en faisant suivre d'une hydrogénation du produit de réaction ainsi obtenu, ou bien
e) par réaction d'aldéhydes cinnamiques de formule 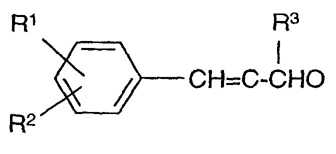 dans laquelle
R¹ à R³ ont les significations indiquées dans la revendication 1, avec des aldéhydes de formule
R⁷R⁸CH-CHO,
Dans laquelle R⁷ et R⁸ ont les significations indiquées dans la revendication 1, et hydrogénation subséquente du produit de réaction,
f) par réaction de benzaldéhydes R¹R²PhCHO, pour lesquels R¹ et R² ont les significations indiquées dans la revendication 1 et Ph représente un groupe phénylène, dans une réaction de Prins, avec le 3-méthyl-3-butén-1-ol, et hydrogénation subséquente de produit de réaction.

10. Compositions de parfums ou d'arômes contenant des composés de formule I selon la revendication 1.

11. Désodorisants contenant des composés de formule I selon la revendication 1.

12. Désodorisants contenant des composés de formule I selon la revendication 1.

13. Désodorisants contenant du 2-méthyl-5-(4-isopropylphényl)-1-pentanol.
